# EUROPEAN PATENT APPLICATION

(11) **EP 3 643 393 A1**
(43) Date of publication of application: **29.04.2020**
(21) Application number: 19196899.9
(22) Date of filing: 12.09.2019
(51) Int. Cl.: B01D 53/22, C07C 7/04, C07C 7/144, C07C 9/08, C07C 11/06

(54) **SYSTEMS AND METHODS OF DEBOTTLENECKING PROPANE FEED**

(30) Priority: 02.10.2018 US 201862740250 P
(71) Applicant: Equistar Chemicals, LP, Houston, Texas 77010 (US); Lyondell Chemical Technology, L.P., Houston, TX 77010 (US)
(72) Inventor: Harry, Carl D., Houston, TX Texas 77010 (US); Fox, Rob D., Houston, TX Texas 77010 (US); Keyvani, Majid, Houston, TX Texas 77010 (US)
(74) Representative: LyondellBasell

(57) **Abstract**

Systems and methods for debottlenecking propane feed, including a propylene separation system and C3 distillation column are provided herein. In some embodiments, the method includes providing a dry feed stream including propane and propylene to a propylene separation system including a membrane configured to separate the C3 feed stream into a retentate stream and a permeate stream, and providing at least a portion of the permeate stream and at least a portion of the propylene discharge stream to a propylene refrigeration system.

## Description

### CROSS-REFERENCE TO RELATED APPLICATIONS

This application claims the benefit of priority to U.S. Provisional Patent Application No. 62/740,250, filed on October 2, 2018, which is incorporated herein by reference in its entirety.

### FIELD OF THE DISCLOSURE

This disclosure relates to separation systems and methods, and more particularly to propane separation methods and systems.

### BACKGROUND

Feedstock selection may involve a combination of factors that include market prices and availability of feedstocks and products in addition to plant conditions. Feedstocks may include, among other things, ethane, propane, butane, natural gasoline, naphtha, condensate, diesel, and/or gas oil. Olefin production facilities may benefit from the ability to process a wide range of feedstocks from ethane to heavy gas oil. However, it is often difficult to accommodate a wide range of feedstock flexibility in a given olefin facility as changing feedstocks can introduce process limitations, and bottlenecks often occur. In particular, when propane feed is preferred for cracking, one unit prone to a bottleneck may be the C3 splitter where propane and propylene are fractionated in a large distillation tower to produce an overhead of polymer grade propylene and a bottom propane stream which is recycled back for cracking..

Commercial scale separation of olefins (ethylene, propylene and butene(s)) from paraffins (ethane, propane and butane(s)) in the petrochemical industry is accomplished almost exclusively by distillation and is considered highly energy and capital intensive. Consequently, there is incentive to explore alternative separation technologies with lower energy consumption and an opportunity to achieve related reductions in environmental impact from air pollutant and greenhouse gas (GHG) emissions. There is also interest to increase production output without incurring the capital expense of duplicating conventional process equipment. Debottlenecking a propane/propylene splitter can be an expensive proposition, since retrofitting may include upgrading existing equipment or installing new equipment.

Accordingly, improved methods and systems for separating propane and propylene and debottlenecking production systems are needed.

### SUMMARY

In one aspect, a method of debottlenecking a production system is provided including: providing a dry feed stream including propane and propylene to a propylene separation system including a membrane configured to separate the feed stream into a retentate stream and a permeate stream, wherein the retentate stream includes propylene at a concentration lower than the concentration of propylene in the feed stream and the permeate stream includes propylene at a concentration higher than the concentration of propylene in the feed stream and wherein the membrane includes (i) a polymeric material and a metal cation or (ii) a ceramic tube with a zeolite active layer; providing the retentate stream to a distillation column to separate propylene from propane and produce a propylene discharge stream and a propane discharge stream; and providing at least a portion of the permeate stream and at least a portion of the propylene discharge stream to a propylene refrigeration system.

In another aspect, a propylene separation system is provided including: a dry feed stream including propane and propylene; a membrane configured to separate the feed stream into a retentate stream and a permeate stream, wherein the retentate stream includes propylene at a concentration lower than the concentration of propylene in the feed stream and the permeate stream includes propylene at a concentration higher than the concentration of propylene in the feed stream, and wherein the membrane includes (i) a polymeric material and a metal cation or (ii) a ceramic tube with a zeolite active layer; a distillation column configured to receive the retentate stream and separate the retentate stream into a propylene discharge stream and a propane discharge stream; and a liquid propylene refrigeration compressor system including an inlet configured to receive at least a portion of the permeate stream and at least a portion of the propylene discharge stream.

### BRIEF DESCRIPTION OF THE DRAWINGS

The following figures illustrate embodiments of the subject matter disclosed herein. The claimed subject matter may be understood by reference to the following description taken in conjunction with the accompanying figures, in which like reference numerals identify like elements, and in which:
**FIG. 1** is a schematic illustration of a propylene separation system according to an embodiment of the present disclosure.
**FIG. 2** is a scatterplot illustrating the weight percent of propane in a permeate stream over time.
**FIG. 3** is a scatterplot illustrating the percent of propylene recovered and the weight percent of propylene in a permeate stream at various stage cut percentages.

### DETAILED DESCRIPTION

Unless explicitly stated otherwise in defined circumstances, all percentages, parts, ratios, and like amounts used herein are defined by weight.

Further, in this connection, certain features of the process, apparatus, or system according to this disclosure which are, for clarity, described herein in the context of separate embodiments, may also be provided in combination in a single embodiment. Conversely, various features of the process, apparatus, or system according to this disclosure that are, for brevity, described in the context of a single embodiment, may also be provided separately or in any sub-combination.

When describing a range of dimensions, concentrations, and the like, it is the Applicant's intent to disclose every individual number that such a range could reasonably encompass, for example, every individual number that has at least one more significant figure than in the disclosed end points of the range. As an example, when referring to a concentration as between 20 mol.% and 30 mol.%, it is intended to disclose that the concentration can be 20 mol.%, 21 mol.%, 22 mol.%, 23 mol.%, 24 mol.%, 25 mol.%, 26 mol.%, 27 mol.%, 28 mol.%, 29 mol.%, or 30 mol.%, including any subranges or combinations of subranges encompassed in this broader range. Applicant's intent is that these two methods of describing the range are interchangeable. Moreover, when a range of values is disclosed or claimed, Applicant also intends for the disclosure of a range to reflect, and be interchangeable with, disclosing any and all sub-ranges and combinations of sub-ranges encompassed therein. Accordingly, Applicant reserves the right to proviso out or exclude any individual members of any such group, including any sub-ranges or combinations of sub-ranges within the group, or any selection, feature, range, element, or aspect that can be claimed, if for any reason Applicant chooses to claim less than the full measure of the disclosure, for example, to account for a reference that Applicant may be unaware of at the time of the filing of the application. In addition, the ranges set forth herein include their endpoints unless expressly stated otherwise.

The term "about" means that amounts, sizes, formulations, parameters, and other quantities and characteristics are not and need not be exact, but may be approximate and/or larger or smaller, as desired, reflecting tolerances, conversion factors, rounding off, measurement error and the like, and other factors known to those of skill in the art. In general, an amount, size, formulation, parameter or other quantity or characteristic is "about" or "approximate" whether or not expressly stated to be such. The term "about" also encompasses amounts that differ due to different equilibrium conditions for a composition resulting from a particular initial mixture. Whether or not modified by the term "about", the claims include equivalents to the quantities. The term "about" may mean within 10% of the reported numerical value, preferably within 5% of the reported numerical value.

In some aspects, methods of debottlenecking a production system are provided which include providing a dry feed stream including propane and propylene to a propylene separation system including a membrane configured to separate the feed stream into a retentate stream and a permeate stream, wherein the retentate stream includes propylene at a concentration lower than the concentration of propylene in the feed stream and the permeate stream includes propylene at a concentration higher than the concentration of propylene in the feed stream and wherein the membrane includes (i) a polymeric material and a metal cation or (ii) a ceramic tube with a zeolite active layer; providing the retentate stream to a distillation column to separate propylene from propane and produce a propylene discharge stream and a propane discharge stream; and providing at least a portion of the permeate stream and at least a portion of the propylene discharge stream to a propylene refrigeration system.

In some aspects, propylene separation systems are provided including: a dry feed stream including propane and propylene; a membrane configured to separate the feed stream into a retentate stream and a permeate stream, wherein the retentate stream includes propylene at a concentration lower than the concentration of propylene in the feed stream and the permeate stream includes propylene at a concentration higher than the concentration of propylene in the feed stream, and wherein the membrane includes (i) a polymeric material and a metal cation or (ii) a ceramic tube with a zeolite active layer; a distillation column configured to receive the retentate stream and separate the retentate stream into a propylene discharge stream and a propane discharge stream; and a liquid propylene refrigeration compressor system including an inlet configured to receive at least a portion of the permeate stream and at least a portion of the propylene discharge stream.

For example, in some embodiments, the membrane includes a polymeric material and a metal cation. Specifically, in some embodiments, the polymeric material may include a polysaccharide or chitosan. In some embodiments, the metal cation is a silver ion or a copper ion. In some embodiments, the membrane is a facilitated membrane. In some embodiments, the membrane is a facilitated polymeric membrane. In some embodiments, the membrane is a facilitated membrane with silver ion embedded therein. In some embodiments, the membrane is a facilitated membrane with silver nitrate. In some embodiments, the membrane includes an aqueous silver nitrate solution over a spiral wound membrane containing a hydrogel coating. Without intending to be bound by any particular theory, it is believed that the silver ions in the silver nitrate solution within the hydrogel structure form a reversible complex with the double bonds of olefin molecules. Then, any molecules not possessing a double bond are rejected by the membrane, resulting in an excellent selectivity of this membrane.

In some embodiments, the ceramic tube with a zeolite active area may include a porous ceramic support coated with high surface area γ-Al₂O₃, as described in Heng, S. Preparation of Composite Zeolite Membrane Separator/Contactorfor Ozone Water Treatment, Micorporous and Mesoporous Materials 115, 137-146 (2008), which is hereby incorporated herein by reference in its entirety. In some embodiments, the ceramic tube with a zeolite active area is formed by a single hydrothermal synthesis of a zeolite LTA membrane on a ceramic hollow fiber, as is described in Wang, Z. et al., High Performance Zeolite LTA Pervaporation Membranes on Ceramic Hollow Fibers by Dipcoating-Wiping Seed Deposition, Journal of the American Chemical Society 131, 6910-6911 (2009), which is hereby incorporated herein by reference in its entirety. In some embodiments, the ceramic tube with a zeolite active area may include a porous polymer hollow fiber with embedded zeolite crystals surrounded by an outer zeolite membrane layer, as described in High-Performance Zeolite NaA Membranes on Polymer-Zeolite Composite Hollow Fiber Supports 131, 17056-17057 (2009), which is hereby incorporated herein by reference in its entirety. In some embodiments, the ceramic tube with a zeolite active area may include a faujasite-type zeolite membrane, as described in Separation of Propylene/Propane Mixtures Using Faujasite-Type Zeolite Membranes; Ind. Eng. Chem. Res. 2005, 44, 226-230, which is hereby incorporated herein by reference in its entirety.

In some embodiments, the propylene refrigeration system includes one or more compressors. For example, in some embodiments, the propylene refrigeration system may include one or more compressors configured to compress propylene to a pressure of at least about 50 psi, for example about 50 psi, about 75 psi, about 100 psi, about 125 psi, about 150 psi, about 175 psi, about 200 psi, and any ranges therebetween. In some embodiments, the propylene refrigeration system has a temperature of no more than 0°F, for example a temperature of about 0°F, about - 10°F, about -20°F, about -30°F, about -40°F, about -50°F, about-60°F, about -70°F, or any ranges therebetween. In some embodiments, the propylene refrigeration system may be a closed loop refrigeration system. In some embodiments, the propylene refrigeration system may be an existing propylene refrigeration system in an olefin plant.

In some embodiments, the permeate stream may be transferred to the refrigeration system without passing through a compressor. For example, in some embodiments the permeate stream may have a pressure of 10-20 psig and may be added to a refrigeration system having a pressure of from about 250-300 psig. Advantageously, transferring the permeate stream to the refrigeration system without first passing the stream through a compressor can result in significant cost savings, both by potentially eliminating the capital expense of a gas compressor, and by eliminating the cost of operating a gas compressor. Further, even when a compressor is used to transfer the permeate stream to the refrigeration system, there is a refrigeration load shift when ethane feed is replaced with propane feed, so there is a natural availability of compressor capacity when propane cracking becomes favored over ethane cracking, which may advantageously result in substantial cost savings.

In some embodiments, the feed stream contains at least 50 mol.% propylene, for example about 50 mol.% propylene, about 55 mol.% propylene, about 60 mol.% propylene, about 65 mol.% propylene, about 70 mol.% propylene, about 75 mol.% propylene, about 80 mol.% propylene, about 85 mol.% propylene, about 90 mol.% propylene, about 95 mol.% propylene, about 96 mol.% propylene, about 97 mol.% propylene, about 98 mol.% propylene, about 99 mol.% propylene, about 99.5 mol.% propylene, or any ranges therebetween. In some embodiments, the permeate stream comprises at least 90 mol.% propylene. In some embodiments, the permeate stream comprises at least 99.5 mol.% propylene.

In some embodiments, the method offers relatively high recovery of propylene in the permeate stream. For example, in some embodiments, the selectivity of the membrane is such that at least about 20% of the propylene in the feed stream is recovered in the permeate stream, for example about 20%, about 25%, about 30%, about 35%, about 40%, about 45%, about 50%, about 55%, about 60%, about 65%, about 70%, about 75%, about 80%, about 85%, about 90%, or any ranges therebetween. In some embodiments, at least about 25% of the propylene in the feed stream is recovered in the permeate stream. In some embodiments, at least about 40% of the propylene in the feed stream is recovered in the permeate stream.

In some embodiments, the feed stream has a pressure of about 315 psia and a temperature of about 155°F, and the permeate stream has a pressure of about 340 psia. In some embodiments, the feed stream has a pressure of about 630 psia and a temperature of 220°F.

### Illustrated Embodiments

**FIG. 1** is a schematic illustration of a propylene separation system **100** according to an embodiment of the present disclosure. In this system **100**, a C3 feed stream **101** is provided to a propylene separation system **103**. The propylene separation system **103** includes a membrane **105** configured to separate the C3 feed stream **101** into a retentate stream **107** and a permeate stream **109**. The retentate stream **107** is a propane-heavy stream, while the permeate stream **109** is a propylene-heavy stream. That is, the retentate stream **107** includes propylene at a concentration lower than the concentration of propylene in the feed stream **101** and the permeate stream **109** comprises propylene at a concentration higher than the concentration of propylene in the C3 feed stream **101**.

The retentate stream **107** is then fed to a C3 distillation column **111**, which is configured to separate the retentate stream **107** into a propane stream **113** and a propylene stream **115**. That is, the retentate stream **107** includes propylene at a concentration lower than the concentration of propylene in the propylene stream **115** and the propane stream **113** comprises propane at a concentration higher than the concentration of propane in the retentate stream **107**.

Next, the permeate stream **109** and the propylene stream **115** are combined to create a combined propylene stream **117**. This combined propylene stream **117** is then fed to a compressor **119** to create a condensed propylene stream **121**. This condensed propylene stream **121** is then fed to a propylene refrigeration system **123**.

### EXAMPLES

The present disclosure is further illustrated by the following examples, which are not to be construed in any way as imposing limitations upon the scope thereof. On the contrary, it is to be clearly understood that resort may be had to various other aspects, embodiments, modifications, and equivalents thereof which, after reading the description herein, may suggest themselves to one of ordinary skill in the art without departing from the spirit of the present invention or the scope of the appended claims. Thus, other aspects of this invention will be apparent to those skilled in the art from consideration of the specification and practice of the invention disclosed herein.

### Example 1: Separation at Varying Feed Pressures

First, a polymeric membrane was used to separate propane from propylene and the results were observed. A mixed C3 stream containing 80 wt.% propylene and 20 wt.% propane was fed to the membrane unit at a rate of 400 cc/min at 65 psig feed pressure and ambient temperature. A permeate flow of 250 cc/min was measured, and the permeate stream contained 99.67 wt.% propylene. The system was operated for over 4 hours, and the weight percent of propane in the permeate stream was regularly measured. As shown in **FIG. 2**, the weight percent of propane in the permeate stream reached an initial peak shortly after starting the experiment at about 0.33 wt.%, before decreasing to about 0.23 wt.%.

Next, a second polymeric membrane was used to separate propane from propylene and the results were observed. A mixed C3 stream containing 80 wt.% propylene and 20 wt.% propane was fed to the membrane unit at a rate of 400 cc/min at 120 psig feed pressure and ambient temperature. A permeate flow was measured, and the permeate stream contained 99.72 wt.% propylene. As shown in **FIG. 2**, the weight percent of propane in the permeate stream reached an initial peak shortly after starting the experiment at about 0.27 wt.%, before decreasing to about 0.26 wt.%.

Thus, by increasing the pressure of the feed stream, the permeate flow increased, while maintaining a comparable purity of propylene in the permeate stream.

### Example 2: Varying the Percent Stage Cut

Using a polymeric membrane, the ratio of the volumetric flow of permeate to the volumetric flow of the feed stream (the "percent stage cut") was varied from about 30% to about 80%. The feed stream contained 70 wt.% propylene and 30 wt.% propane, and the percent of the propylene recovered (% olefin recovery) in the permeate stream, and the weight percent of propylene in the permeate stream was measured. These results are shown in **FIG. 3**.

### Example 3: Purification of a Propylene/Propane Stream using a Ceramic Membrane

A feed stream containing 80 wt.% propylene and 20 wt.% propane was fed to a ceramic membrane at an actual volumetric flow rate of 8 SLPM (Standard Liter per minute) liters/min. at 190° F and 127 psig pressure. A permeate stream containing 94.0% propylene was obtained at feed pressure of 127 psig and a permeate pressure of 14 psig (188° F).

### Example 4: Purification of Propylene/Propane Stream Using Ceramic Membrane

A feed stream containing 80 wt.% propylene and 20 wt.% propane was fed to a ceramic membrane at an actual volumetric flow rate of 4 SLPM at 160° F, 152 psig and a permeate pressure of 13 psig. A permeate stream containing 92.63% propylene was obtained. The residue stream contained a mixture containing 51% propane and 49% propylene.

The present disclosure is described above with reference to numerous aspects and embodiments, and specific examples. Equivalents and certain variations of the disclosed embodiments will suggest themselves to those skilled in the art in light of the above detailed description, which are within the intended scope of the appended claims.

## Claims

1. A method of debottlenecking a production system comprising:
providing a dry feed stream comprising propane and propylene to a propylene separation system comprising a membrane configured to separate the feed stream into a retentate stream and a permeate stream, wherein the retentate stream comprises propylene at a concentration lower than the concentration of propylene in the feed stream and the permeate stream comprises propylene at a concentration higher than the concentration of propylene in the feed stream and wherein the membrane comprises (i) a polymeric material and a metal cation or (ii) a ceramic tube with a zeolite active layer;
providing the retentate stream to a distillation column to separate propylene from propane and produce a propylene discharge stream and a propane discharge stream; and
providing at least a portion of the permeate stream and at least a portion of the propylene discharge stream to a propylene refrigeration system.

2. The method of claim 1, wherein the membrane comprises a polymeric material and a metal cation.

3. The method of claim 2, wherein the polymeric material of the membrane comprises a polysaccharide or chitosan.

4. The method of claim 2, wherein the metal cation of the membrane is selected from a silver ion or a copper ion.

5. The method of claim 1, wherein the membrane comprises a facilitated membrane.

6. The method of claim 1, wherein the propylene refrigeration system comprises one or more compressors, and wherein the propylene refrigeration system has a temperature of about - 40°F.

7. The method of claim 1, wherein the feed stream comprises at least 60 mol.% propylene.

8. The method of claim 1, wherein the feed stream has a pressure of about 315 psia and a temperature of about 155°F and the permeate stream has a pressure of about 340 psia.

9. A propylene separation system comprising:
a dry feed stream comprising propane and propylene;
a membrane configured to separate the feed stream into a retentate stream and a permeate stream, wherein the retentate stream comprises propylene at a concentration lower than the concentration of propylene in the feed stream and the permeate stream comprises propylene at a concentration higher than the concentration of propylene in the feed stream, and wherein the membrane comprises (i) a polymeric material and a metal cation or (ii) a ceramic tube with a zeolite active layer;
a distillation column configured to receive the retentate stream and separate the retentate stream into a propylene discharge stream and a propane discharge stream; and
a liquid propylene refrigeration compressor system comprising an inlet configured to receive at least a portion of the permeate stream and at least a portion of the propylene discharge stream.

10. The propylene separation system of claim 9, wherein the membrane comprises a polymeric material and a metal cation.

11. The propylene separation system of claim 9, wherein the polymeric material of the membrane comprises a polysaccharide or chitosan.

12. The propylene separation system of claim 9, wherein the metal cation of the membrane is selected from a silver ion or a copper ion.

13. The propylene separation system of claim 9, wherein the feed stream comprises at least 60 mol.% propylene.

14. The propylene separation system of claim 9, wherein the feed stream has a pressure of about 315 psia and a temperature of about 155°F and the permeate stream has a pressure of about 340 psia.

15. The propylene separation system of claim 9, wherein the feed stream has a pressure of about 630 psia and a temperature of about 220°F.
